# EUROPEAN PATENT APPLICATION

(11) **EP 4 113 537 A1**
(43) Date of publication of application: **04.01.2023**
(21) Application number: 21315120.2
(22) Date of filing: 30.06.2021
(51) Int. Cl.: G16H 50/20, G16H 30/40

(54) **METHODS FOR TRAINING A PREDICTION MODEL, OR FOR PROCESSING AT LEAST A PRE-CONTRAST IMAGE DEPICTING A BODY PART PRIOR TO AN INJECTION OF CONTRAST AGENT USING SAID PREDICTION MODEL**

(71) Applicant: Guerbet, 93420 Villepinte (FR)
(72) Inventor: STANCANELLO, Joseph, 91190 GIF SUR YVETTE (FR)
(74) Representative: Regimbeau

(57) **Abstract**

The present invention relates to a method for processing at least a pre-contrast image depicting a body part prior to an injection of contrast agent, the method being characterized in that it comprises the implementation, by a data processor (11b) of a second server (1b), of steps of:
(a) Obtaining said pre-contrast image;
(b) Determining, by application of a prediction model to said pre-contrast image candidate value(s) of at least one injection parameter of said injection of contrast agent, such that the sequence of said pre-contrast image and at least a contrast image depicting said body part during injection of contrast agent in accordance with the determined candidate value(s) of said injection parameter(s) minimizes a radiation dose.

## Description

### FIELD OF THE INVENTION

The field of this invention is that of machine learning.

More particularly, the invention relates to methods for training an artificial intelligence (machine or deep learning) model (e.g. convolutional neural network) for processing at least a pre-contrast image, and for using such artificial intelligence model, in particular for the optimization of injection protocol parameters to lower radiation doses.

### BACKGROUND OF THE INVENTION

Contrast agent are substances used to increase the contrast of structures or fluids within the body in medical imaging.

They usually absorb or alter external radiations emitted by the medical imaging device. In x-rays, contrast agents enhance the radiodensity in a target tissue or structure.

X-ray mammography is a non-contrast enhanced modality of first line investigation for breast cancer in women. Some studies highlighted that up to 30% of lesions are missed in non-contrast enhanced mammographies. Contrast-enhanced mammography (CEM) has shown an increase in sensitivity and, to a lesser extent, to specificity with respect to standard plain mammography, and could be conducted following a standard plain mammography that yielded abnormal or inconclusive findings. CEM shows promise as a valuable tool to identify and diagnose breast cancer, and it offers a fast and non-expensive alternative to magnetic resonance imaging (MRI).

In CEM, an iodinated contrast agent is injected intravenously to the patient and rapid repeated imaging is performed in order to obtain a temporal sequence of breast images with very high sensitivity. On the other hand, CEM is based on a dynamic x-ray-based imaging acquisition, involving radiation exposure (higher than a plain mammography), in contrast to MRI. Recent clinical guidelines suggest to lower exposure, as a precautionary consideration.. This is in alignment with ALARA principle, i.e. radiation dose As Low As Reasonably Achievable.

Each image of a CEM may involve a lower radiation dose than a plain mammography, but CEM requires a plurality of images to be acquired. It is possible to decrease the radiation dose of each image by tweaking image acquisition parameters (settings of the medical imaging device, such as kVp (kilovoltage peak), spatial resolution, etc.) but it decreases the image quality. Note that it might be possible to « restore » low quality images at the diagnostic quality for instance by Al based algorithms, but it is not yet reliable.

Alternatively, it can be attempted to minimize the total radiation dose (instead of considering the radiation dose of each image) while preserving the diagnostic value of the acquired CEM series, but is very difficult because of the interplay among said acquisition parameters, injection parameters (in particular the amount of contrast agent, contrast injection speed, and time delay to acquisition) and physiological parameters (for example cardiac output, patient specific hemodynamics parameters). While the first two groups are controllable variables, the third group cannot be changed but represents a given, fix set of parameters depending on individual patient physiology.

Therefore, it is very challenging to have a "personalized contrast injection protocol" that will achieve the lowest radiation dose while preserving the diagnostic value of the acquired CEM series.

There is consequently still a need for a new method for determining optimal parameters for medical imaging with injection of contrast agent.

### SUMMARY OF THE INVENTION

For these purposes, the present invention provides according to a first aspect a method a method for processing at least a pre-contrast image depicting a body part prior to an injection of contrast agent, the method being characterized in that it comprises the implementation, by a data processor of a second server, of steps of:
(a) Obtaining said pre-contrast image;
(b) Determining, by application of a prediction model to said pre-contrast image candidate value(s) of at least one injection parameter of said injection of contrast agent, such that the sequence of said pre-contrast image and at least a contrast image depicting said body part during injection of contrast agent in accordance with the determined candidate value(s) of said injection parameter(s) minimizes a radiation dose.

Preferred but non limiting features of the present invention are as it follows:
Step (a) also comprises obtaining value(s) of at least one context parameter of said pre-contrast image; said prediction model using said at least one context parameter as input at step (b) such that said contrast image has the same value(s) of context parameter(s) as the pre-contrast image:

Said context parameter(s) is (are) physiological parameter(s) and/or acquisition parameter(s).

Said injection parameter(s) comprises an injection duration prior to the . acquisition of the contrast image.

Said pre-contrast image is acquired by an x-ray medical imaging device connected to the second server, said radiation dose being inflicted by the x-ray medical imaging device to said body part.

Said pre-contrast and contrast images are mammographies.

The method comprises a step (c) of providing said determined candidate value(s) of said injection parameter(s) to the x-ray medical device, and obtaining in response the contrast image depicting said body part during injection of contrast agent in accordance with the determined candidate value(s) of said injection parameter(s), acquired by said x-ray medical imaging device.

Said contrast image, candidate value(s) are respectively a i-th contrast image and i-th candidate value(s), with i>0, the method comprising a step (d) of determining i+1-th candidate value(s) of the injection parameter by application of the prediction model to at least the i-th contrast image, such that the sequence of said pre-contrast image, each j-th contrast image, 0<j≤i, and the i+1-th contrast image depicting said body part during injection of contrast agent in accordance with the determined i+1-th candidate value(s) of said injection parameter(s) minimizes the radiation dose.

Step (d) comprises combining the i-th contrast image with the pre-contrast image and/or at least one j-th contrast image, 0<j<i, into a combined image, the prediction model being applied to the combined image.

The method comprises a step (e) of providing said i+1-th candidate value(s) of said injection parameter(s) to the x-ray medical device, and obtaining in response a i+1-th contrast image depicting said body part during injection of contrast agent in accordance with the i+1-th candidate value(s) of said injection parameter(s), acquired by said medical imaging device.

The method comprises recursively iterating steps (d) and (e) so as to obtain the sequence of successive contrast images.

Said prediction model comprises a Convolutional Neural Network, CNN.

According to a second aspect, the invention provides a method for training a prediction model, the method being characterized in that it comprises the implementation, by a data processor of a first server, for each of a plurality of training pre-contrast images from a base of training pre-contrast or contrast images respectively depicting a body part prior to and during an injection of contrast agent, each image being at least associated to reference value(s) of at least one injection parameter of said injection of contrast agent, of a step of determining candidate value(s) of said injection parameter(s) by application of the prediction model to said training pre-contrast image, such that the sequence of said training pre-contrast image and at least a contrast image depicting said body part during injection of contrast agent in accordance with the determined candidate value(s) of said injection parameter(s) minimizes a radiation dose; and verifying if the sequence of said theoretical contrast image and the contrast image minimize the radiation dose.

Preferred but non limiting features of the present invention are as it follows: the training pre-contrast or contrast images are organized in the base into sequences corresponding to the same injection, verifying for a training pre-contrast images if the sequence of said theoretical contrast image and the contrast image minimize the radiation dose comprises comparing the determined candidate value(s) of said injection parameter(s) with the reference value(s) of the injection parameter(s) associated to the training contrast image following said pre-contrast image in the same sequence.

According to a third and a fourth aspect the invention provides a computer program product comprising code instructions to execute a method according to the first aspect for training at least a prediction model, or according to the second aspect for processing at least a pre-contrast image depicting a body part prior to an injection of contrast agent; a computer-readable medium, on which is stored a computer program product comprising code instructions for executing a method according to the first aspect for training at least a prediction model, or according to the second aspect for processing at least a pre-contrast image depicting a body part prior to an injection of contrast agent.

### BRIEF DESCRIPTION OF THE DRAWINGS

The above and other objects, features and advantages of this invention will be apparent in the following detailed description of an illustrative embodiment thereof, which is to be read in connection with the accompanying drawings wherein:
- figure 1 illustrates an example of architecture in which the methods according to the invention is performed;
- figure 2 illustrates an embodiment of the methods according to the invention.

### DETAILED DESCRIPTION OF A PREFERRED EMBODIMENT

### Architecture

Two complementary aspects of the present invention are proposed:
- a method for training at least a prediction model for processing at least a pre-contrast image;
- a method for processing at least a pre-contrast image using the prediction model, advantageously trained according to the previous method.

By pre-contrast image, or "plain" image, it is meant an image depicting a given body part (to be monitored) prior to an injection of contrast agent, for a person or an animal. By contrast image it is meant an image depicting said body part during or after the injection of contrast agent.

In other words, if there is a temporal sequence of images, the first one is the pre-contrast image, and each of the following is a contrast image.

In the preferred case of contrast-enhanced mammography (CEM), the body part is a breast, i.e. the (pre-contrast or contrast) images depict inner structures of breasts, in particular to measure breast density and morphologic features. The contrast images further highlight increased blood flow related to angiogenesis.

The (pre-contrast or contrast) images are either directly acquired, or derived from images directly acquired, by a medical imaging device using ionizing radiations, in particular an X-ray medical imaging device such as a mammograph as explained, but also for instance an X-ray rotational scanner.

The acquisition of a said image may involve the injection of a contrast agent such as iodinated contrast agent.

The prediction model is an artificial intelligence (Al) algorithm, e.g. a neural network (NN - and in particular a convolutional neural network, CNN) but possibly a Support Vector Machine (SVM), Random Forests (RF), etc., trained using machine learning (ML) or deep learning (DL) algorithms. In the following description we will take the preferred example of a CNN (referred to as prediction CNN), but the invention is not limited to this embodiment, in particular Al algorithms of different natures could be used.

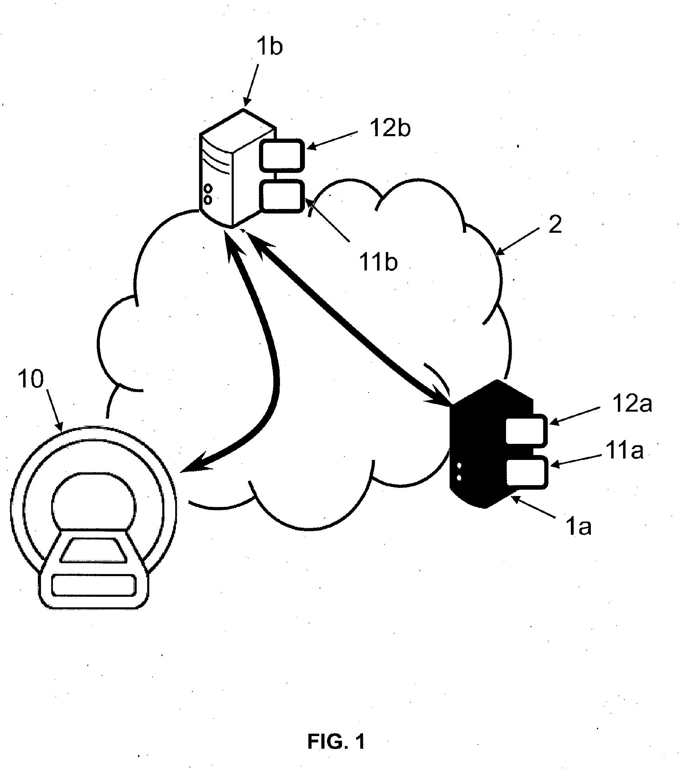

The above-mentioned methods are implemented within an architecture such as illustrated in **Figure 1****,** by means of a first and/or second server 1a, 1b. The first server 1a is the training server (implementing the training method) and the second server 1b is a processing server (implementing the processing method). It is fully possible that these two servers may be merged.

Each of these servers 1a, 1b is typically remote computer equipment connected to an extended network 2 such as the Internet for data exchange. Each one comprises data processing means 11a, 11b of processor type (in particular the data processor 11a of the first server 1a have strong computing power, since learning is long and complex compared with ordinary use of the trained models), and optionally storage means 12a, 12b such as a computer memory e.g. a hard disk. The second server 1b may be connected to one or more medical imaging devices 10 as client equipment, to provide images to be processed, and receive back parameters.

Note that it is supposed that the imaging device 10 comprises an injector to perform the injection of contrast agent, said injector applying the injection parameters.

The memory 12a of the first server 1a stores a training database i.e. a set of images referred to as training images (as opposed to so-called inputted images that precisely are sought to be processed). Each image of the database could be pre-contrast or contrast. Note that images corresponding the same injection (i.e. forming a sequence) are associated into sequences. Each image/set of images is also associated to the corresponding parameters (at least one injection parameter, and preferably at least one context parameter chosen among a physiological parameter and/or acquisition parameter), and possibly to a quality level. Said quality level is in particular selected among a predefined plurality of possible quality levels.

In a preferred embodiment, there are only two quality levels: "good image quality", i.e. an image with diagnostic image quality; and "poor image quality", i.e. an image with non-diagnostic image quality. Note that there may be more quality levels. The quality levels of training images are typically obtained by consensus of a given number of expert radiologists.

### Obtaining the pre-contrast image

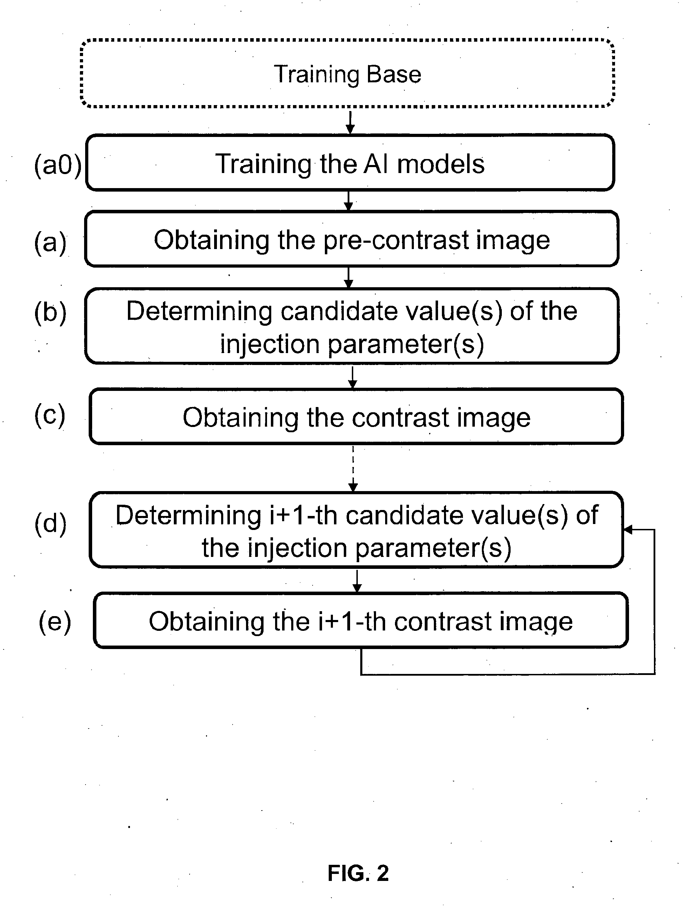

As represented by **figure 2****,** the method for processing at least a pre-contrast image starts with a step (a) of obtaining said pre-contrast image to be processed, preferably from a medical imaging device 10 connected to the second server 1a which has acquired said pre-contrast image.

Preferably, step (a) may also comprise obtaining value(s) of at least one context parameter of said pre-contrast image, typically physiological parameter(s) and/or acquisition parameter(s). As explained, physiological parameters are parameters related to the individual patient whose body part is depicted by the images (e.g. cardiac output, patient specific hemodynamics parameters, etc.), and acquisition parameters are related to the medical imaging device 10 (i.e. settings of the medical imaging device 10, such as kVp, mA, exposure time, etc.).

Indeed, the present invention proposes to consider acquisition parameters as also not variable (like the physiological parameters) and focus only on injection protocol parameters (amount of contrast agent, contrast injection speed, time delay to acquisition, etc.) which are to be optimized. Preferably, the injection protocol parameter to be optimized is the time delay to acquisition, i.e. the delay for the next image acquisition. For instance, by assuming that the injection immediately starts after the pre-contrast image is acquired, there is generally approximately two minutes before acquiring the first contrast image. Any image acquired before the right moment could be a useless radiation dose.

Thus, in the present invention, the radiation dose delivered to the patient (during the acquisition of the sequence) is astutely decreased by acquiring the minimal number of images at given time points.

Note that this step (a) may be implemented by the data processor 11b of the second server 1b and/or by the medical imaging device 10 if for instance it associates the parameters to the pre-contrast image.

### Prediction model

In a main step (b), implemented by the data processor 11b of the second server 1b, the prediction model is applied to said pre-contrast image.

In more details, step (b) aims at determining candidate value(s) of the at least one injection parameter, i.e. the output of said prediction model is the value(s) of the injection parameter(s).

The so-called candidate values are potentially optimized values, such that the sequence of said pre-contrast image and at least a contrast image depicting said body part during injection of contrast agent in accordance with the determined candidate value(s) of said injection parameter(s) minimize a radiation dose

In other words, the prediction model predicts the values of the injections parameters which should lead to the realization of a contrast image minimizing the radiation dose.

It is to be understood that:
(1) by "radiation dose" it is meant the total dose absorbed by the body part (for instance expressed in mSv) over the whole examination, i.e. for the entire sequence of said pre-contrast image and said at least a contrast image, the model does not have for instance to compute a dose specifically associated to each image.
(2) The radiation dose that will be observed in reality may actually be lowerable. In other words, the determined candidate values are the optimal ones according to the model, but not always the absolute optimal ones. In the context of the present invention it is sufficient (and much easier, which is important if the process is intended to be performed in real time) that the prediction model simply use the radiation dose as the optimization criterion, but it will not guarantee that the radiation dose cannot be further lowered. To rephrase again "to minimize a radiation dose" is not a result to be achieved but a criterion to be optimized as much as possible.

The prediction model advantageously uses the at least one context parameter as input at step (b), such that said theoretical contrast image further has the same value(s) of context parameter(s) as the pre-contrast image. In other words, the prediction model uses as input, the pre-contrast image and the value(s) of said context parameter(s) of said pre-contrast image, and outputs the candidate value(s) of the injection parameter(s).

Indeed, as explained, for simplifying the process, each context parameters (acquisition parameter(s) and/or physiological parameter(s)) is supposed fixed and only values of injection protocol parameters are determined. Therefore, the pre-contrast images and the subsequent contrast images are supposed to have the same values of the context parameters.

Note that predicting candidate value(s) of the injection parameter(s) minimizing the dose may be done according to several paradigms.

In a first embodiment, said prediction model could be a direct estimation model if the training database is suitably constructed, i.e. if the sequences of training images are supposed to minimize the radiation dose. In other words, for a training pre-contrast image, the value(s) of injection parameter(s) associated to the next contrast image of the same sequence can be considered as "expected" value(s) of injection parameter(s) minimizing the radiation dose (i.e. ground truth) for this pre-contrast image. Thus, the prediction model takes as input the inputted pre-contrast and estimates the associated value(s) of injection parameter(s). Suitable CNN are well known to the skilled person, see for example VGG-16 or AlexNet.

In a second embodiment, the prediction model uses a property of the radiation dose: image quality and radiation dose are linked by specific equations in x-ray imaging. In particular, image quality increases almost linearly within some radiation dose ranges and the overall relationship can be modelled by a logistic function, see for instance the document *General equations for optimal selection of diagnostic image acquisition parameters in clinical X-ray imaging, by Xiaoming Zheng.*

Therefore, instead of assuming that the database is suitably constructed, the database could be labelled with a quality level and let the model learn all the correspondences by means of a data-driven empirical approach. In other words, the prediction model is provided by said equations linking image quality and radiation dose, and it actually determines the candidate value(s) of the injection parameter(s), such that the theoretical contrast image depicting said body part during injection of contrast agent in accordance with the determined candidate value(s) of said injection parameter(s) is expected to present a target quality level corresponding to a . minimal radiation dose.

In other words, the prediction model predicts the values of the injections parameters which should lead to the realization of a contrast image with the target quality level that will actually minimize the radiation dose. To rephrase, said "target" quality is defined as a function of the radiation dose.

Note that the contrast image here is referred to as "theoretical" as:
(1) Said image is generally never acquired. Indeed, the prediction model merely assess the possibility of existence of said image. Note that in some embodiment the theoretical image might be simulated but it is not compulsory.
(2) The "real" contrast image depicting said body part during injection of contrast agent in accordance with the determined candidate value(s) of said injection parameter(s) may actually not present said target quality level, because there is always a probability that the prediction does not come true.

A contrast image presenting a target quality level can be construed as an inverse problem. Indeed, it is actually easier to train a "test" model outputting an estimated quality of contrast image from the pre-contrast image and the candidate value(s) of the injection parameter(s) than a direct prediction model. The idea is thus to predict the candidate value(s) of the injection parameter(s) by trial-and-error, i.e. to iteratively test several possible values until reaching the target quality level. The tested values can be randomly selected, or according to a pattern.

The "test" model may be a two-step model (i.e. two sub-models) which (1) simulates the theoretical contrast image (or even generates it) from the pre-contrast image and the candidate value(s) of the injection parameter(s) (and the set context parameter(s)), and (2) estimates the quality of the simulated theoretical contrast image: The first sub-model may be a generator model for example based on a GAN (Generative adversarial network) trained for generating synthetic contrast images (a discriminator module of the GAN tries to distinguish original contrast images from a database from synthetic contrast images). The second sub-model is a classification model, that could be any Al algorithm, in particular a CNN, taking as input the contrast image and determining its quality level. CNN for classification of images are well known to the skilled person, see for example VGG-16 or AlexNet.

A classification model is very efficient, as the quality level can be chosen among a predefined plurality of possible quality levels as alternate classes.

Note that any other possible model able to predict the candidate value(s) of the injection parameter(s) is suitable.

### Use of the candidate value(s) of the injection parameter(s)

The method preferably comprises a step (c) of providing said determined candidate value(s) of said injection parameter(s) to the medical device 10.

Therefore, a contrast image depicting said body part during injection of contrast agent in accordance with the determined candidate value(s) of said injection parameter(s) can be acquired by said medical imaging device 10. Because of the use of the candidate value(s) of said injection parameter(s), the sequence of the pre-contrast image and the contrast image is expected to minimize the radiation dose inflicted to the patient.

Step (c) advantageously further comprises obtaining in response (at data processor 11b of a second server 1b, from the imaging device 10) the acquired contrast image, in particular for iterating the process.

Indeed, the present method can be applied to static contrast acquisition (e.g. two images, the pre-contrast image and one contrast image, for example portal or delayed phase), but also to dynamic contrast acquisition (as it is generally the case for CEM) involving a sequence of contrast images, i.e. a plurality of contrast images depicting said body part during injection of contrast agent.

In a preferred embodiment, the present method could be performed recursively for ensuring that the complete sequence of the pre-contrast image and each contrast image minimizes the radiation dose.

In the context of a sequence of contrast image, each contrast image and candidate value(s) is respectively referred to as i-th contrast image and i-th candidate value(s), with i>0. In order, the pre-contrast image is acquired, then the first contrast image, the second contrast image, etc.

The present method preferably comprises a step (d) of determining the i+1-th candidate value(s) of said injection parameter(s): the sequence of the pre-contrast image and each subsequent contrast image (i.e. including each j-th contrast image, 0<j≤i, and the i+1-th contrast image depicting said body part during injection of contrast agent in accordance with the determined i+1-th candidate value(s) of said injection parameter(s)) still minimizes the radiation dose. Each j-th contrast image, 0<j≤i, is already available, only the i+1-th contrast image is yet to be acquired. In other words, in step (d), knowing the pre-contrast image and each j-th contrast image up to the i-th one, the candidate value(s) of said injection parameter(s) for the i+1-th contrast image are optimized to continue minimizing the radiation dose.

Again, the injection protocol parameter to be optimized is preferably the time delay to acquisition, i.e. the delay between the acquisition of the i-th contrast image and the acquisition of the i+1-th contrast image.

Note that the step (d) can be seen as a generic version of step (b), with step (b) as the "0-th" iteration of the step (d), and then the step (d) repeated as many times as there are further contrast images after the first one.

Similarly, the method preferably comprises a step (e) of providing said i+1-th candidate value(s) of said injection parameter(s) to the medical device 10, which is similar to step (c). A i+1-th contrast image depicting said body part during injection of contrast agent in accordance with the i+1-th candidate value(s) of said injection parameter(s) can acquired by said medical imaging device 10. Step (f) advantageously further comprises obtaining in response (at data processor 11b of a second server 1b, from the imaging device 10) the acquired i+1-th contrast image, in particular again for recursively minimizing the radiation dose.

Then a new occurrence of step (d) may be performed, i.e. determining i+2-th candidate value(s) of the injection parameter(s), etc.

Indeed, the method advantageously comprises recursively iterating steps (d) to (e) so as to obtain a sequence of successive contrast images. Note that said sequence may be interrupted (i.e. no more image is acquired) for instance if a fixed time observational window (e.g. 2 minutes) is over, or if a specific metric (e.g. difference, mutual information, etc.) between two consecutive images is below/above a given threshold.

The prediction model may be only applied to the i-th contrast image, but preferably, step (e) comprises combining the i-th contrast image with the pre-contrast image and/or at least one j-th contrast image, 0<j<i, into a combined image (even preferably combining the i-th contrast image with the pre-contrast image and each j-th contrast image, 0<j<i, i.e. all the i+1 previously acquired images), the prediction model being applied to the combined image.

In other words, the information from previously acquired image may be taken into account when determining the i+1-th candidate value(s) so as to refine this determination and improve the chances to have a really minimal radiation zone over the whole sequence.

There might be several implementations for combining pre-contrast/contrast images:
- the images may be aggregated into a hyper-stack with associated parameters;
- The images may be summed, averaged, etc.
- the combined image could be based on subtraction of the images to highlight the differences;
- a combination thereof, for instance a hyper-stack comprising the i-th contrast image and the subtraction of several previous images;
- etc.

### Training method

In a second aspect, there is proposed a training method, implemented by the data processor 11a of the first server 1a. Said method trains the prediction model, for processing at least a pre-contrast image depicting a body part prior to an injection of contrast agent.

By training, it is meant the determination of the optimal values of parameters and weights for this Al model.

Note that the model used in the processing method is preferably trained according to the present training method, hence referred to at step (a0) in the figure 2. Note that alternatively the model may be directly taken "off the shelf" with preset values of parameters and weights.

Said training method is similar to the previously described processing method, but is iteratively performed on training images of the training database, i.e. a base of training pre-contrast or contrast images respectively depicting a body part prior to and during an injection of contrast agent, each image being associated to reference value(s) of at least one injection parameter of said injection of contrast agent and organized into sequences corresponding to the same injection. Training images are preferably further associated to a reference quality level.

In particular, the training method comprises, for each of a plurality of training pre-contrast images from the training base, a step of determining candidate value(s) of said injection parameter(s) by application of the prediction model to said training pre-contrast image, such that the sequence of said training pre-contrast image and at least a contrast image depicting said body part during injection of contrast agent in accordance with the determined candidate value(s) of said injection parameter(s) minimizes a radiation dose; and verifying if the sequence of said theoretical contrast image and the contrast image minimize the radiation dose.

The training may be direct. In particular, if the sequence of the training pre-contrast and the corresponding training contrast image is known to minimize the radiation dose, minimizing the radiation dose for a given training pre-contrast image is equivalent to produce a "real" contrast image as close as possible to the training contrast image following said pre-contrast image in the same sequence, i.e. equivalent to predict candidate value(s) of said injection parameter(s) as close as possible to the reference value(s) of the injection parameter(s) associated to said training contrast image. Thus, said prediction of model can be verified by comparing the determined candidate value(s) and reference value(s) of the injection parameter(s).

Alternatively, as explained the prediction model may be trained so as to predict candidate value(s) of the injection parameter(s), such that the theoretical contrast image depicting said body part during injection of contrast agent in accordance with the determined candidate value(s) of said injection parameter(s) is expected to present a target quality level corresponding to a minimal radiation dose. The training may again be direct (if there is an identified contrast image presenting said target quality level corresponding to the minimal radiation dose belonging to the same sequence as said training pre-contrast image, said "theoretical" contrast image can be verified by comparing the determined candidate value(s) and reference value(s) of the injection parameter(s) of said identified training image), or there may be two sub-models that are independently trained on the training base:
- a generator model for simulating or generating the theoretical contrast image depicting said body part during injection of contrast agent in accordance with the determined candidate value(s) of said injection parameter(s), for instance trained so as to produce theoretical contrast images as realistic as possible; and
- a classification model for determining the quality level of a contrast image, for instance trained so as to determine for training contrast images candidate quality level as close as possible the reference quality level of the training contrast image.

Any training protocol adapted to the Al types of the prediction models known to a skilled person may be used.

### Computer program product

In a third and fourth aspect, the invention provides a computer program product comprising code instructions to execute a method (particularly on the data processor 11a, 11b of the first or second server 1a, 1b) according to the second aspect of the invention for training at least a prediction model, or a method according to the first aspect of the invention for processing at least a pre-contrast image depicting a body part prior to an injection of contrast agent, and storage means readable by computer equipment (memory of the first or second server 1a, 1b) provided with this computer program product.

## Claims

1. A method for processing at least a pre-contrast image depicting a body part prior to an injection of contrast agent, the method being **characterized in that** it comprises the implementation, by a data processor (11b) of a second server (1b), of steps of:
(a) Obtaining said pre-contrast image;
(b) Determining, by application of a prediction model to said pre-contrast image candidate value(s) of at least one injection parameter of said injection of contrast agent, such that the sequence of said pre-contrast image and at least a contrast image depicting said body part during injection of contrast agent in accordance with the determined candidate value(s) of said injection parameter(s) minimizes a radiation dose.

2. A method according to claim 1, wherein step (a) also comprises obtaining value(s) of at least one context parameter of said pre-contrast image; said prediction model using said at least one context parameter as input at step (b) such that said contrast image has the same value(s) of context parameter(s) as the pre-contrast image.

3. The method according to claim 2, wherein said context parameter(s) is (are) physiological parameter(s) and/or acquisition parameter(s).

4. The method according to one of claims 1 to 3, wherein said injection parameter(s) comprises an injection duration prior to the acquisition of the contrast image.

5. The method according to one of claims 1 to 4, wherein said pre-contrast image is acquired by an x-ray medical imaging device (10) connected to the second server (1a), said radiation dose being inflicted by the x-ray medical imaging device (10) to said body part.

6. The method according to claim 5, wherein said pre-contrast and contrast images are mammographies.

7. The method according to one of claims 5 and 6, comprising a step (c) of providing said determined candidate value(s) of said injection parameter(s) to the x-ray medical device (10), and obtaining in response the contrast image depicting said body part during injection of contrast agent in accordance with the determined candidate value(s) of said injection parameter(s), acquired by said x-ray medical imaging device (10).

8. The method according to claim 7, wherein said contrast image, candidate value(s) are respectively a i-th contrast image and i-th candidate value(s), with i>0, the method comprising a step (d) of determining i+1-th candidate value(s) of the injection parameter by application of the prediction model to at least the i-th contrast image, such that the sequence of said pre-contrast image, each j-th contrast image, 0<j≤i, and the i+1-th contrast image depicting said body part during injection of contrast agent in accordance with the determined i+1-th candidate value(s) of said injection parameter(s) minimizes the radiation dose.

9. The method according to claim 8, wherein step (d) comprises combining the i-th contrast image with the pre-contrast image and/or at least one j-th contrast image, 0<j<i, into a combined image, the prediction model being applied to the combined image.

10. The method according to one of claims 8 and 9, comprising a step (e) of providing said i+1-th candidate value(s) of said injection parameter(s) to the x-ray medical device (10), and obtaining in response a i+1-th contrast image depicting said body part during injection of contrast agent in accordance with the i+1-th candidate value(s) of said injection parameter(s), acquired by said medical imaging device (10).

11. The method according to claim 10, comprising recursively iterating steps (d) and (e) so as to obtain the sequence of successive contrast images.

12. The method according to one of claims 1 to 11, wherein said prediction model comprises a Convolutional Neural Network, CNN.

13. A method for training a prediction model, the method being **characterized in that** it comprises the implementation, by a data processor (11a) of a first server (1a), for each of a plurality of training pre-contrast images from a base of training pre-contrast or contrast images respectively depicting a body part prior to and during an injection of contrast agent, each image being at least associated to reference value(s) of at least one injection parameter of said injection of contrast agent, of a step of determining candidate value(s) of said injection parameter(s) by application of the prediction model to said training pre-contrast image, such that the sequence of said training pre-contrast image and at least a contrast image depicting said body part during injection of contrast agent in accordance with the determined candidate value(s) of said injection parameter(s) minimizes a radiation dose; and verifying if the sequence of said theoretical contrast image and the contrast image minimize the radiation dose.

14. The method according to claim 13, wherein the training pre-contrast or contrast images are organized in the base into sequences corresponding to the same injection, verifying for a training pre-contrast images if the sequence of said theoretical contrast image and the contrast image minimize the radiation dose comprises comparing the determined candidate value(s) of said injection parameter(s) with the reference value(s) of the injection parameter(s) associated to the training contrast image following said pre-contrast image in the same sequence.

15. Computer program product comprising code instructions to execute a method according to one of claims 1 to 14 for training at least a prediction model, or for processing at least a pre-contrast image depicting a body part prior to an injection of contrast agent, when said program is executed on a computer
